# NEW EUROPEAN PATENT SPECIFICATION

(11) **EP 0 076 563 B2**
(45) Date of publication and mention of the opposition decision: **10.05.1995**
(45) Mention of the grant of the patent: 11.11.1987
(21) Application number: 82304154.6
(22) Date of filing: 06.08.1982
(51) Int. Cl.: A61K 7/16

(54) **Toothpaste**
Zahnpasta
Pâte dentifrice

(30) Priority: 06.10.1981 US 309072
(43) Date of publication of application: 13.04.1983
(73) Proprietor: BEECHAM INC., West Paterson New Jersey 07424 (US)
(72) Inventor: Stier, Roger Edwin, Clifton New Jersey 07011 (US); Minczuk, Andrew, Passaic New Jersey 07055 (US)
(74) Representative: Lockwood, Barbara Ann

(56) References cited:
- DE-A- 2 053 109
- DE-A- 2 619 936
- DE-B- 1 937 001
- FR-A- 2 113 128
- FR-A- 2 242 968
- GB-A- 891 027
- GB-A- 962 757
- GB-A- 1 130 929
- GB-A- 1 435 624
- US-A- 2 905 364
- US-A- 3 175 731
- US-A- 3 980 767
- Modern Packaging 34 (6), pp. 98-100
- Marketing Week, Jan. 23, 1981

## Description

This invention relates to a toothpaste in the form of three or more separate phases at least two of which are transparent or translucent gels.

U.K. Patent Specification No. 1,271,944 discloses toothpastes of this general type, wherein the main body of the paste is a transparent or translucent gel, and the secondary bodies contain cleaning and polishing agents.

We have now found that excellent cleaning and polishing properties, together with high visual attractiveness, can be obtained with a toothpaste in which the main body is an opaque material containing a cleaning or polishing agent, and the secondary bodies are transparent or translucent gels.

According to the present invention there is provided a multi-phase toothpaste which, both inside a collapsible container and when extruded therefrom through a nozzle, comprises a paste matrix and a plurality of secondary paste bodies embedded in and discrete from the paste matrix, characterised in that from 60 to 80% w/w of the total paste consists of the paste matrix, which is an opaque material containing a cleaning or polishing agent, and from 20 to 40% w/w of the total paste consists of the secondary bodies which include at least two transparent or translucent gel bodies of different colours and which are in the form of gel stripes only partly contained within the matrix so that a portion of the surface of the stripes is exposed at the surface of the toothpaste.

The colours of the secondary bodies may serve to distinguish between bodies containing different active ingredients, and also to provide visual distinctiveness. For example, one of the secondary bodies may contain an anti-stain or mouth freshening ingredient, while another secondary body, of different colour from the first, may contain an anti-plaque agent. The composition of the paste matrix and secondary material should be such as to minimise diffusion or other undesirable interaction between the matrix and material. To this end, it is preferred to match the pH values of the different phases as closely as possible. It has also been found that enhanced stability can be achieved if the rheological properties of the phases are substantially equal.

Substances which are normally incompatible with the secondary-bodies, e.g. substances that would adversely affect or destroy the light transmitting properties of the secondary bodies, may be included in the paste matrix as a major component without significantly affecting these properties. For example, dentally acceptable abrasives, such as chalk, dicalcium phosphate dihydrate, calcium pyrophosphate, alumina, PVC powder and polymethylmethacrylate, as well as other substances which are incompatible with the secondary bodies, may be included in the paste matrix without affecting the stability of the secondary bodies.

In a preferred embodiment of the invention, the toothpaste has a paste matrix of an opaque white colour and two or more gel stripes of different colours extending longitudinally along the outside of the matrix.

In a particularly preferred embodiment, there are four gel stripes spaced equidistantly around the toothpaste, and suitably two diametrically opposed stripes are of one colour and two are of a different colour.

As used herein, 'colour' means any visually perceivable colour, such as white, red, blue etc., or the absence of colour, i.e. 'water-white' (totally clear and transparent).

The secondary bodies in the toothpaste of the present invention are preferably aqueous gels thickened with natural or synthetic thickeners, such as a natural or synthetic gum, carageenates, alginates, cellulose ethers and esters and, carboxyvinyl polymers. Where the gels are aqueous, it is desirable to include a humectant, such as glycerine, sorbitol, propylene glycol or a polyethylene glycol. Active ingredients may be included in the matrix or secondary bodies or both.

In accordance with conventional practice, the toothpaste of the invention may include materials such as detergents, sweeteners, flavouring agents, anti-caries agents and dentally acceptable abrasive material. For example, the toothpaste of the invention may comprise 15 to 75% dentally acceptable abrasive, 10 to 80% humectant, 0.25 to 5% detergent, and 0 to 2% sweeteners and flavouring agents, together with water and thickening agents to form the gel or gels. Colouring agents will be included to provide the desired colours. Other agents that may be included in the toothpaste are therapeutic agents for plaque or calculus reduction. Examples of such agents are disclosed in U.S. Patent No. 4,022,880.

The multi-phase toothpaste of the invention may be readily prepared by feeding the several phases through separate tubes of a bundle of tubes. Where the secondary bodies are stripes, the matrix phase is fed through a central tube and the secondary phases through tubes arranged around the central tube. The bundle of tubes is inserted into a toothpaste container and gradually moved upwardly relative to the container as the container is filled.

The filling device will generally have a casing enclosing the bundle of tubes, and a main outlet orifice via the central tube, and to this end the central tube is connected to a dispensing device, usually a pumping device, for feeding the matrix material to and through the central tube. The secondary bodies are dispensed in a similar fashion through one or more secondary tubes parallel to and surrounding the central tube.

A suitable apparatus for dispensing a multi-phase toothpaste in this manner is based on that described in U.K. Patent Specification No. 962,757. The apparatus of Specification No. 962,757 may be modified so that each secondary tube may be connected to a separate source of gel material. This arrangement will then permit the dispensing of two or more secondary bodies of different colour. It has been already used to make a toothpaste having a transparent or translucent jelly main body and opaque stripes constituting 10% by weight of the toothpaste. See US-A-3,980,767.

The following Examples illustrate the invention. All parts and proportions are by weight unless otherwise stated.

### Example

A toothpaste having a white paste matrix and four gel stripes is prepared from the following ingredients:

| (a) Gel Phases | % w/w | |
|---|---|---|
| | Blue Gel | Red Gel |
| 70% Sorbitol solution | 50.00 | 52.00 |
| Precipitated silica | 10.00 | 12.00 |
| Polyethylene Glycol | 3.00 | 4.00 |
| Sodium Carboxymethyl Cellulose | 1.30 | 1.30 |
| Sodium Lauryl Sulphate | 1.00 | 1.00 |
| Spearmint Essence | 0.40 | 0.50 |
| Saccharin | 0.15 | 0.15 |
| Calcium Glycerophosphate | 0.13 | 0.13 |
| Sodium Monofluorophosphate | 0.76 | 0.76 |
| Blue dye | qs | - |
| Yellow dye | qs | - |
| Red Dye | - | qs |
| Water | to 100.00 | 100.00 |

| (a) White Paste Matrix | % w/w |
|---|---|
| 70% Sorbitol solution | 20.00 |
| Polyethylene Glycol | 3.00 |
| Precipitated silica | 10.00 |
| Sodium Carboxymethyl Cellulose | 1.30 |
| Sodium Lauryl Sulphate | 1.00 |
| Spearmint Essence | 1.10 |
| Saccharin | 0.27 |
| Calcium Glycerophosphate | 0.13 |
| Sodium Monofluorophosphate | 0.76 |
| Titanium Dioxide | 1.15 |
| Calcium Carbonate | 25.00 |
| Water | to 100.00 |

The blue and red gels, and white paste matrix, are each separately prepared from the above ingredients, and are charged into an open toothpaste tube, the paste matrix being dispensed from a central tube and the red and blue gels being dispensed from tubes spaced circumferentially around the central tube, according to the method and apparatus described in U.K. Patent Specification No. 962,757.

The red gel is dispensed from two tubes located diametrically opposite to the central tube, and the blue gel is dispensed from two other tubes located diametrically opposite the central tube. A toothpaste having a white paste matrix and alternating red and blue stripes is obtained.

## Claims

1. A multi-phase toothpaste which, both inside a collapsible container and when extruded therefrom through a nozzle, comprises a paste matrix and a plurality of secondary paste bodies embedded in and discrete from the matrix, characterised in that from 60 to 80% w/w of this total paste consists of the paste matrix, which is an opaque material containing a cleaning or polishing agent, and from 20 to 40% w/w of the total paste consists of the secondary bodies which include at least two transparent or translucent gel bodies of different colours and which are in the form of gel stripes partly contained within the matrix so that a portion of the surface of the stripes is exposed at the surface of the toothpaste.

2. A toothpaste according to claim 1 in which the paste matrix comprises an opaque white paste with two or more gel stripes extending longitudinally along the outside of the matrix.

3. A toothpaste according to claim 2 in which four gel stripes are spaced equidistantly around the paste matrix, two diametrically opposed stripes being of one colour and two being of a different colour.

4. A collapsible tube, or other container, containing a toothpaste according to any one of claims 1 to 3, the paste matrix and secondary bodies being so arranged within the tube or container that when the tube or container is collapsed the secondary bodies will be discharged as stripes within the paste matrix.

5. A method of making a toothpaste according to claim 1, in which the paste matrix is fed throughout a central tube and the secondary bodies are fed through tubes arranged around the central tube so as to apply the secondary bodies to the paste matrix as the paste matrix leaves the outlet orifice of the central tube.

## Patentansprüche

1. Mehrphasenzahnpaste, die sowohl innerhalb eines Quetschbehälters als auch, wenn sie durch eine Düse daraus extrudiert wird, eine Pastenmatrix und eine Vielzahl von sekundären Pastenkörpern aufweist, die in der und von der Pastenmatrix getrennt eingebettet sind, dadurch gekennzeichnet, daß 60 bis 80 % G/G dieser gesamten Zahnpaste aus der Pastenmatrix, die ein opakes Material ist, das ein Reinigungs- oder Poliermittel enthält, und 20 bis 40 % G/G der gesamten Zahnpaste aus den sekundären Körpern bestehen, die zumindest zwei transparente oder transluzente Gelkörper mit verschiedenen Farben inkludieren, und die in Form von Gelstreifen vorliegen, die teilweise innerhalb der Matrix enthalten sind, so daß ein Teil der Oberfläche der Streifen an der Oberfläche der Zahnpaste exponiert ist.

2. Zahnpaste nach Anspruch 1 , bei der die Pastenmatrix eine opake weiße Paste umfaßt, wobei sich zwei oder mehr Gelstreifen der Länge nach längs der Außenseite der Matrix erstrecken.

3. Zahnpaste nach Anspruch 2, bei der vier Gelstreifen in gleichem Abstand rund um die Pastenmatrix angeordnet sind, wobei zwei diametral gegenüberliegende Streifen eine Farbe und zwei eine andere Farbe aufweisen.

4. Quetschtube oder ein anderer Behälter enthaltend eine Zahnpaste nach einem der Ansprüche 1 bis 3, wobei die Pastenmatrix und die sekundären Körper innerhalb der Tube oder des Behälters so angeordnet sind, daß, wenn die Tube oder der Behälter zusammengedrückt wird, die sekundären Körper als Streifen innerhalb der Pastenmatrix abgegeben werden.

5. Verfahren zum Herstellen einer Zahnpaste nach Anspruch 1, worin die Pastenmatrix durch ein mittleres Rohr und die sekundären Körper durch Rohre zugeführt werden, die rund um das mittlere Rohr angeordnet sind, um die sekundären Körper auf die Pastenmatrix aufzubringen, wenn die Pastenmatrix die Auslaßöffnung des mittleren Rohres verläßt.

## Revendications

1. Pâte dentifrice à plusieurs phases qui, à la fois à l'intérieur d'un récipient pliable et lorsqu'elle est extrudée hors de celui-ci par un ajutage, comprend une matrice de pâte et un grand nombre de corps de pâte secondaires noyés dans la matrice de pâte et distincts de celle-ci, caractérisée en ce que de 60 à 80% en p/p de cette pâte totale consistent en matrice de pâte, qui est une matière opaque contenant un agent de nettoyage ou de polissage, et que de 20 à 40% en p/p de la pâte totale consistent en corps secondaires qui comprennent au moins deux corps de gel transparents ou translucides de couleur différente et qui sont sous la forme de bandes de gel contenues en partie à l'intérieur de la matrice de telle sorte qu'une partie de la surface des bandes est exposée à la surface de la pâte dentifrice.

2. Pâte dentifrice suivant la revendication 1, dans laquelle la matrice de pâte comprend une pâte blanche opaque avec deux ou plusieurs bandes de gel se prolongeant longitudinalement le long de l'extérieur de la matrice.

3. Pâte dentifrice suivant la revendication 2, dans laquelle quatre bandes de gel sont disposées à équidistance autour de la matrice de pâte, deux bandes diamétralement opposées étant d'une couleur et deux bandes étant d'une couleur différente.

4. Tube pliable ou autre récipient, contenant une pâte dentifrice suivant l'une quelconque des revendications 1 à 3, la matrice de pâte et les corps secondaires étant disposés à l'intérieur du tube ou du récipient de telle sorte que lorsque le tube ou le récipient est pressé, les corps secondaires sortent sous forme de bandes à l'intérieur de la matrice de pâte.

5. Procédé de fabrication d'une pâte dentifrice suivant la revendication 1, dans lequel la matrice de pâte est introduite à travers un tube central et les corps secondaires sont envoyés à travers des tubes disposés autour du tube central de façon à appliquer les corps secondaires sur la matrice de pâte alors que la matrice de pâte quitte l'orifice de sortie du tube central.
